(19)

Europäisches
Patentamt
European
Patent Office
Office européen
des brevets

(11)     **EP 1 761 274 B1**

(12)                    **EUROPEAN PATENT SPECIFICATION**

(45) Date of publication and mention
    of the grant of the patent:
    **02.09.2009  Bulletin 2009/36**

(51) Int Cl.:
    *A61K 38/48* (2006.01)    *A61P 7/04* (2006.01)

(21) Application number: **05756838.8**

(22) Date of filing: **21.06.2005**

(86) International application number:
    **PCT/EP2005/052890**

(87) International publication number:
    **WO 2005/123118 (29.12.2005 Gazette 2005/52)**

(54) **FACTOR VIIA OR FACTOR VIIA EQUIVALENTS FOR PREVENTING OR ATTENTUATING HAEMORRHAGE GROWTH, AND/OR OEDEMA GENERATION FOLLOWING INTRACEREBRAL HAEMORRHAGE (ICH)**

FAKTOR VIIA ODER FAKTOR VIIA ÄQUIVALTENEN ZUR PRÄVENTION ODER ABSCHWÄCHUNG DES WACHSTUMS VON HÄMORRHAGIEN UND/ODER DER ÖDEMERZEUGUNG NACH INTRAZEREBRALER HÄMORRHAGIE (ICH)

FACTOR VIIA OU D'EQUIVALENTS DU FACTEUR VIIA POUR LA PREVENTION OU L'ATTENUATION D'UNE EVOLUTION HEMORRAGIQUE ET/OU DE LA FORMATION D'OEDEME APRES UNE HEMORRAGIE INTRACEREBRALE (HIC)

(84) Designated Contracting States:
    **AT BE BG CH CY CZ DE DK EE ES FI FR GB GR HU IE IS IT LI LT LU MC NL PL PT RO SE SI SK TR**

(30) Priority: **21.06.2004  DK 200400972
    23.06.2004  DK 200400981**

(43) Date of publication of application:
    **14.03.2007  Bulletin 2007/11**

(73) Proprietor: **Novo Nordisk Health Care AG
    8050 Zürich (CH)**

(72) Inventors:
  • **BRUN, Nikolai Constantin
    DK-2200 Copenhagen N (DK)**
  • **ERHARDTSEN, Elisabeth
    DK-2900 Herlev (DK)**
  • **SKOLNICK, Brett E.
    Philadelphia, Pennsylvania 19130 (US)**

(74) Representative: **Nilsson, Karin Norvin
    Novo Nordisk A/S,
    Corporate Patents,
    Novo Allé
    2880 Bagsvaerd (DK)**

(56) References cited:
    **EP-A- 1 216 709    WO-A-98/58661
    WO-A-02/062376**

• **MAJUMDAR G; SAVIDGE G F: "Recombinant factor VIIa for intracranial haemorrhage in a Jehovah's Witness with severe haemophilia A and factor VIII inhibitors" BLOOD COAGULATION & FIBRINOLYSIS: AN INTERNATIONAL JOURNAL IN HAEMOSTASIS AND THROMBOSIS, vol. 4, no. 6, December 1993 (1993-12), pages 1031-1033, XP009055682**
• **DIRINGER M N; AIYAGARI V; SHACKLEFORD A M; BRODY D L: "Use of recombinant factor VIIa in patients with warfarin-associated intracranial hemorrhage" BLOOD, vol. 102, no. 11, 16 November 2003 (2003-11-16), page 109B, XP009055681**
• **CHUANSUMRIT AMPAIWAN ET AL.: "Outcome of intracranial hemorrhage in infants with congenital factor VII deficiency" JOURNAL OF THE MEDICAL ASSOCIATION OF THAILAND, vol. 85, no. Suppl4, November 2002 (2002-11), pages S1059-S1064, XP009055678**
• **WONG W-Y ET AL: "CLINICAL EFFICACY AND RECOVERY LEVELS OF RECOMBINANT FVIIa (NovoSeven) IN THE TREATMENT OF INTRACRANIAL HAEMORRHAGE IN SEVERE NEONATAL FVII DEFICIENCY" HAEMOPHILIA, BLACKWELL SCIENCE, OXFORD, GB, vol. 6, no. 1, January 2000 (2000-01), pages 50-54, XP002178100 ISSN: 1351-8216**

**Description**

**FIELD OF THE INVENTION**

The invention relates to the manufacture of a medicament for preventing or minimising the severity of complications in ICH patients.

**BACKGROUND OF THE INVENTION**

Haemostasis is a complex physiological process which ultimately results in the arrest of bleeding. This is dependent on the proper function of three main components: blood vessels (especially the endothelial lining), coagulation factors, and platelets. Once a haemostatic plug is formed, the timely activation of the fibrinolytic system is equally important to prevent further unnecessary haemostatic activation. Any malfunction of this system (due to a reduced number, or molecular dysfunction, of the haemostatic components or increased activation of the fibrinolytic components) may lead to clinical bleeding such as, e.g., haemorrhagic diathesis of varying severity.

In most physiological situations, haemostasis is triggered by the interaction of circulating activated coagulation factor VII (FVIIa) with tissue factor (TF) subsequent to exposure of TF at the site of an injury. Endogenous FVIIa becomes proteolytically active only after forming a complex with TF. Normally, TF is expressed in the deep layers of the vessel wall and is exposed following injury. This ensures a highly localized activation of coagulation and prevents disseminated coagulation. TF also seems to exist in a non-active form, so-called encrypted TF. The regulation of encrypted versus active TF is still unknown.

Intracerebral haemorrhage (ICH) is a neurologic condition that occurs spontaneous and results in blood collecting in the intraparenchymal brain tissue. The results of an ICH have been demonstrated to result in significant morbidity and mortality. In recent years ICH has been shown to increase in volume in the hours following the initial Insult. This occurs in at approximately 38% of patients suffering from ICH. The reason for the increase is unclear, but it is thought to be either through a continuous oozing of the original haematoma or through a complex process of rebleeds.

Days after the initial insult a zone of oedema can be identified on CT scans ... surrounding the blood in the haematoma. The mechanism for oedema generation is also poorly understood but may be due to a combination of an inflammatory reaction in the tissue surrounding the clot as well as a direct mass effect of the clot exerting pressure on surrounding brain tissue. The impact of the isolated oedema can be significant but not evaluated in the context of significant haemorrhage but can have effects on the volume of compromised brain tissue after an ICH which has been estimated to be up to 3 times the actual volume of the haematoma. The importance of overall effected tissue volume would appear to be one of the strongest predictors of outcome after ICH. Thus there is clinical interest in reducing any haemorrhage expansion and in reducing and/or minimizing the total lesion volume (blood and resulting oedema).

Thus, there is a need in the art for improved compositions for the acute treatment of ICH, as well as for prevention and attenuation of later complications that result from ICH and from conventional modalities that are used to treat patients with ICH.

**SUMMARY OF THE INVENTION**

The invention provides the use of a first coagulation agent comprising Factor VIIa, or a Factor VIIa equivalent, for manufacture of a medicament for preventing or attenuating one or more complications of intracerebral haemorrhage (ICH) in a patient, wherein said patient has not been diagnosed with a congenital bleeding disorder and has not previously been treated with anticoagulation therapies.

In one aspect, said patient has experienced spontaneous or traumatic ICH.

In a second aspect, the administered amount of Factor VIIa or Factor VIIa equivalent comprises at least about 40 $\mu$g/kg, such as at least about 80 $\mu$g/kg, such as at least about 120 $\mu$g/kg.

In a third aspect, the Factor VIIa or Factor VIIa equivalent is administered within about 24 hours of the occurrence of the ICH.

In a fourth aspect, the Factor VIIa or Factor VIIa equivalent is administered within about 4 hours of the occurrence of the ICH.

In an fifth aspect, the medicament further comprises a second coagulation agent; wherein the amounts of said first and second coagulation agents together are effective in said preventing or attenuating ICH. This second coagulation agent may be a coagulation factor, selected, for example, from any one of the group consisting of Factor VIII, Factor IX, Factor V, Factor XI, Factor XIII, a Factor VII or Factor VIIa. The second coagulation agent may also be an antifibrinolytic agent, selected from the group consisting of PAI-1, aprotinin, $\epsilon$-aminocaproic acid, tranexamic acid and combinations of any of the foregoing.

In a further aspect, said preventing or attenuating effect results in one or more of a reduction in the number of days an ICH patient is hospitalized following ICH and a reduction in the risk of death in an ICH patient.

In another aspect, the invention provides, the use of Factor VIIa or a Factor VIIa equivalent for the manufacture of a medicament for reducing the number of days of hospitalization of an ICH patient, including days in the Intensive Care Unit (ICU), bed confinement, and/or Quality of Life as measured by the European Quality of Life Scale (EuroQOL), or similar instruments, in the period from start of treatment (SOT) to day 90, preferably day 15 following the start of treatment or for reducing the risk of death in an ICH patient. In one embodiment, (i) an amount of 40, 80 or 160 μg/kg of Factor VIIa or Factor VIIa equivalent is administered to the patient at the start of treatment as a slow single bolus

## DETAILED DESCRIPTION OF THE INVENTION

The present invention provides compositions that can be used advantageously to prevent or attenuate haemorrhage growth, and/or oedema generation following ICH, which patient may experience subsequent to their injury and/or as a result of medical interventions that may be used to treat their injuries. The methods are carried out by administering, to an ICH patient, a Factor VIIa or a Factor VIIa equivalent, in a manner that is effective for preventing or attenuating haemorrhage growth, oedema formation as well as one or more complications related to ICH. A manner effective for preventing or attenuating haemorrhage growth, oedema formation and the subsequent complications may comprise administering a predetermined amount of Factor VIIa or a Factor VIIa equivalent, and/or utilizing a particular dosage regimen, formulation, mode of administration, combination with other treatments, and the like. The efficacy of the methods of the invention in reducing haemorrhage growth, oedema formation or in preventing complications of ICH may be assessed using one or more conventional imaging methods (e.g., CT, MRI scanning) or by use of parameters that evaluate complications (see below). Complications that may be prevented by the use according to the current invention, or whose severity may be attenuated, include, without limitation, haemorrhage growth, oedema generation, and decreased quality of life including death caused by one or more of these syndromes.

### *Patient selection:*

Patients who may benefit from the use according to the present invention include, without limitation, patients who have suffered from spontaneous or traumatic ICH. Spontaneous ICH includes patients suffering an intracerebral bleed usually associated with the occurrence of advanced age, hypertension, or deposition of amyloid in the cerebral vasculature. ICH usually results from the rupture of a single vessel causing extensive damage to the surrounding brain tissue adjacent to the damaged vessel. Traumatic ICH may be associated with accidents resulting from e.g. motor vehicle accidents or fall from a height. The resulting contusion to the head may lead to the rupture of one or more intracerebral or extracerebral (but intracranial) vessels. Many intracranial (but extracerebral) bleedings are evacuated surgically already in the acute phase, whereas the intracerebral lesions more often are inaccessible to direct evacuation as the evacuation itself would cause significant damage to the brain tissue.

Bleeding refers to extravasation of blood from any component of the circulatory system and encompasses any bleeding (including, without limitation, excessive, uncontrolled bleeding, i.e., haemorrhaging) in connection with ICH. In one series of embodiments, the excessive bleeding is caused by spontaneous ICH; in another it is caused by traumatic ICH.

The use according to the present invention can be applied advantageously to any patient who has suffered spontaneous or traumatic ICH that, if left untreated, would result in a significant growth of the haemorrhage and in associated oedema and/or complications.

Patients treated according to the invention do not suffer from a congenital bleeding disorder.

### *Factor VIIa and Factor VIIa equivalents:*

In practicing the present invention, any Factor VIIa or equivalent may be used that is effective in preventing complications when administered to an ICH patient. In some embodiments, the Factor VIIa is human Factor VIIa, as disclosed, e.g., in U.S. Patent No. 4,784,950 (wild-type Factor VII). The term "Factor VII" is intended to encompass Factor VII polypeptides in their uncleaved (zymogen) form, as well as those that have been proteolytically processed to yield their respective bioactive forms, which may be designated Factor VIIa. Typically, Factor VII is cleaved between residues 152 and 153 to yield Factor VIIa.

Factor VIIa equivalents include, without limitation, Factor VII polypeptides that have either been chemically modified relative to human Factor VIIa and/or contain one or more amino acid sequence alterations relative to human Factor VIIa. Such equivalents may exhibit different properties relative to human Factor VIIa, including stability, phospholipid binding, altered specific activity, and the like.

In one series of embodiments, a Factor VIIa equivalent includes polypeptides that exhibit at least about 10%, preferably at least about 30%, more preferably at least about 50%, and most preferably at least about 70%, of the specific biological activity of human Factor VIIa. For purposes of the invention, Factor VIIa biological activity may be quantified by measuring the ability of a preparation to promote blood clotting using Factor VII-deficient plasma and thromboplastin, as described, e.g., in U.S. Patent No. 5,997,864. In this assay, biological activity is expressed as the reduction in clotting time relative to a control sample and is converted to "Factor VII units" by comparison with a pooled human serum standard containing 1 unit/ml Factor VII activity. Alternatively, Factor VIIa biological activity may be quantified by (i) measuring the ability of Factor VIIa or a Factor VIIa equivalent to produce of Factor Xa in a system comprising TF embedded in a lipid membrane and Factor X. (Persson et al., J. Biol. Chem. 272:19919-19924, 1997); (ii) measuring Factor X hydrolysis in an aqueous system (see, Example 5 below); (iii) measuring the physical binding of Factor VIIa or a Factor VIIa equivalent to TF using an instrument based on surface plasmon resonance (Persson, FEBS Letts. 413:359-363, 1997) and (iv) measuring hydrolysis of a synthetic substate by Factor VIIa and/or a Factor VIIa equivalent.

Examples of factor VII equivalents include, without limitation, wild-type Factor VII, L305V-FVII, L305V/M306D/D309S-FVII, L305I-FVII, L305T-FVII, F374P-FVII, V158T/M298Q-FVII, V158D/E296V/M298Q-FVII, K337A-FVII, M298Q-FVII, V158D/M298Q-FVII, L305V/K337A-FVII, V158D/E296V/M298Q/L305V-FVII, V158D/E296V/M298Q/K337A-FVII, V158D/E296V/M298Q/L305V/K337A-FVII, K157A-FVII, E296V-FVII, E296V/M298Q-FVII, V158D/E296V-FVII, V158D/M298K-FVII, and S336G-FVII, L305V/K337A-FVII, L305V/V158D-FVII, L305V/E296V-FVII, L305V/M298Q-FVII, L305V/V158T-FVII, L305V/K337A/V158T-FVII, L305V/K337A/M298Q-FVII, L305V/K337A/EZ96V-FVII, L305V/K337A/V158D-FVII, L305V/V158D/M298Q-FVII, L305V/V158D/E296V-FVII, L305V/V158T/M298Q-FVII, L305V/V158T/E296V-FVII, L305V/EZ96V/M298Q-FVII, L305V/V158D/E296V/M298Q-FVII, L305V/V158T/E296V/M298Q-FVII, L305V/V158T/K337A/M298Q-FVII, L305V/V158T/E296V/K337A-FVII, L305V/V158D/K337A/M298Q-FVII, L305V/V158D/EZ96V/K337A-FVII, L305V/V158D/E296V/M298Q/K337A-FVII, L305V/V158T/E296V/M298Q/K337A-FVII, S314E/K316H-FVII, S314E/K316Q-FVII, S314E/L305V-FVII, S314E/K337A-FVII, S314E/V158D-FVII, S314E/E296V-FVII, S314E/M298Q-FVII, S314E/V158T-FVII, K316H/L305V-FVII, K316H/K337A-FVII, K316H/V158D-FVII, K316H/E296V-FVII, K316H/M298Q-FVII, K316H/V158T-FVII, K316Q/L305V-FVII, K316Q/K337A-FVII, K316Q/V158D-FVII, K316Q/E296V-FVII, K316Q/M298Q-FVII, K316Q/V158T-FVII, S314E/L305V/K337A-FVII, S314E/L305V/V158D-FVII, S314E/L305V/E296V-FVII, S314E/L305V/M298Q-FVII, S314E/L305V/V158T-FVII, S314E/L305V/K337A/V158T-FVII, S314E/L305V/K337A/M298Q-FVII, S314E/L305V/K337A/E296V-FVII, S314E/L305V/K337A/V158D-FVII, S314E/L305V/V158D/M298Q-FVII, S314E/L305V/V158D/E296V-FVII, S314E/L305V/V158T/M298Q-FVII, S314E/L305V/V158T/E296V-FVII, S314E/L305V/E296V/M298Q-FVII, S314E/L305V/V158D/E296V/M298Q-FVII, S314E/L305V/V15ST/E296V/M298Q-FVII, S314E/L305V/V158T/K337A/M298Q-FVII, S314E/L305V/V158T/E296V/K3377A-FVII, S314E/L305V/V158D/K337A/M298Q-FVII, S314E/L305V/V158D/E296V/K337A-FVII, S314E/L305V/V158D/E296V/M298Q/K337A-FVII, S314E/L305V/V158T/E296V/M298Q/K337A-FVII, K316H/L305V/K337A-FVII, K316H/L305V/V158D-FVII, K316H/L305V/E296V-FVII, K316H/L305V/M298Q-FVII, K316H/L305V/V158T-FVII, K316H/L305V/K337A/V158T-FVII, K316H/L305V/K337A/M298Q-FVII, K316H/L305V/K337A/E296V-FVII, K316H/L305V/K337A/V158D-FVII, K316H/L305V/V158D/M298Q-FVII, K316H/L305V/V158D/E296V-FVII, K316H/L305V/V158T/M298Q-FVII, K316H/L305V/V158T/E296V-FVII, K316H/L305V/E296V/M298Q-FVII, K316H/L305V/V158D/EZ96V/M298Q-FVII, K316H/L305V/V158T/E296V/M298Q-FVII, K316H/L305V/V158T/K337A/M298Q-FVII, K316H/L305V/V158T/E296V/K337A-FVII, K316H/L305V/V158D/K337A/M298Q-FVII, K316H/L305V/V158D/E296V/K337A-FVII, K316H/L305V/V158D/E296V/M298Q/K337A-FVII, K316H/L305V/V158T/E296V/M298Q/K337A-FVII, K316Q/L305V/K337A-FVII, K316Q/L305V/V158D-FVII, K316Q/L305V/E296V-FVII, K316Q/L305V/M298Q-FVII, K316Q/L305V/V158T-FVII, K316Q/L305V/K337A/V158T-FVII, K316Q/L305V/K337A/M298Q-FVII, K316Q/L305V/K337A/E296V-FVII, K316Q/L305V/K337A/V158D-FVII, K316Q/L305V/V158D/M298Q-FVII, K316Q/L305V/V158D/E296V-FVII, K316Q/L305V/V158T/M298Q-FVII, K316Q/L305V/V158T/E296V-FVII, K316Q/L305V/E296V/M298Q-FVII, K316Q/L305V/V158D/E296V/M298Q-FVII, K316Q/L305V/V158T/E296V/M298Q-FVII, K316Q/L305V/V158T/K337A/M298Q-FVII, K316Q/L305V/V158T/E296V/K337A-FVII, K316Q/L305V/V158D/K337A/M298Q-FVII, K316Q/L305V/V158D/E296V/K337A-FVII, K316Q/L305V/V158D/E296V/M298Q/K337A-FVII, and K316Q/L305V/V158T/E296V/M298Q/K337A-FVII.

### Preparations and formulations:

The present invention encompasses therapeutic administration of Factor VIIa or Factor VIIa equivalents, which is achieved using formulations that comprise Factor VIIa preparations. As used herein, a "Factor VII preparation" refers to a plurality of Factor VIIa polypeptides or Factor VIIa equivalent polypeptides, including variants and chemically modified forms, that have been separated from the cell in which they were synthesized, whether a cell of origin or a recombinant cell that has been programmed to synthesize Factor VIIa or a Factor VIIa equivalent.

Separation of polypeptides from their cell of origin may be achieved by any method known in the art, including, without limitation, removal of cell culture medium containing the desired product from an adherent cell culture; centrifugation or filtration to remove non-adherent cells; and the like.

Optionally, Factor VII polypeptides may be further purified. Purification may be achieved using any method known in the art, including, without limitation, affinity chromatography, such as, e.g., on an anti-Factor VII antibody column (see, e.g., Wakabayashi et al., J. Biol. Chem. 261:11097, 1986; and Thim et al., Biochem. 27:7785, 1988); hydrophobic interaction chromatography; ion-exchange chromatography; size exclusion chromatography; electrophoretic procedures (*e.g.*, preparative isoelectric focusing (IEF), differential solubility (*e.g.*, ammonium sulfate precipitation), or extraction and the like. See, generally, Scopes, Protein Purification, Springer-Verlag, New York, 1982; and Protein Purification, J.-C. Janson and Lars Ryden, editors, VCH Publishers, New York, 1989. Following purification, the preparation preferably contains less than about 10% by weight, more preferably less than about 5% and most preferably less than about 1%, of non-Factor VII proteins derived from the host cell.

Factor VII and Factor VII-related polypeptides may be activated by proteolytic cleavage, using Factor XIIa or other proteases having trypsin-like specificity, such as, e.g., Factor IXa, kallikrein, Factor Xa, and thrombin. See, e.g., Osterud et al., Biochem. 11:2853 (1972); Thomas, U.S. Patent No. 4,456,591; and Hedner et al., J. Clin. Invest. 71:1836 (1983). Alternatively, Factor VII may be activated by passing it through an ion-exchange chromatography column, such as Mono Q® (Pharmacia) or the like. The resulting activated Factor VII may then be formulated and administered as described below.

Pharmaceutical compositions or formulations for use in the present invention comprise a Factor VIIa preparation in combination with, preferably dissolved in, a pharmaceutically acceptable carrier, preferably an aqueous carrier or diluent. A variety of aqueous carriers may be used, such as water, buffered water, 0.4% saline, 0.3% glycine and the like. The preparations of the invention can also be formulated into liposome preparations for delivery or targeting to the sites of injury. Liposome preparations are generally described in, e.g., U.S. Patents Nos. 4,837,028, 4,501,728, and 4,975,282. The compositions may be sterilised by conventional, well-known sterilisation techniques. The resulting aqueous solutions may be packaged for use or filtered under aseptic conditions and lyophilised, the lyophilised preparation being combined with a sterile aqueous solution prior to administration.

The compositions may contain pharmaceutically acceptable auxiliary substances or adjuvants, including, without limitation, pH adjusting and buffering agents and/or tonicity adjusting agents, such as, for example, sodium acetate, sodium lactate, sodium chloride, potassium chloride, calcium chloride, etc.

### *Treatment regimen:*

In practicing the present invention, Factor VIIa or the Factor VIIa equivalent may be administered to a patient as a single dose comprising a single-dose-effective amount for preventing haemorrhage growth, and/or oedema formation and/or for treating complications, or in a staged series of doses which together comprise an effective amount for preventing or treating complications. An effective amount of Factor VIIa or the Factor VIIa equivalent (see below) refers to the amount of Factor VIIa or equivalent which, when administered in a single dose or in the aggregate of multiple doses, or as part of any other type of defined treatment regimen, produces a measurable statistical improvement in outcome, as evidenced by at least one clinical parameter associated with ICH and/or its complications (see below). When Factor VIIa equivalents are administered, an effective amount may be determined by comparing the coagulant activity of the Factor VIIa equivalent with that of Factor VIIa and adjusting the amount to be administered proportionately to the predetermined effective dose of Factor VIIa.

Administration of Factor VIIa or a Factor VIIa equivalent according to the present invention is preferably initiated within about 4 hours after occurrence of the ICH such as, e.g., within about 3 hours, within about 2 hours, or within about 1 hour.

Administration of a single dose refers to administration of an entire dose of Factor VIIa or the Factor VIIa equivalent as a slow bolus over a period of less than about 5 minutes. In some embodiments, the administration occurs over a period of less than about 2.5 minutes, and, in some, over less than about 1 min. Typically, a single-dose effective amount comprises at least about 40 μg/kg human Factor VIIa or a corresponding amount of a Factor VIIa equivalent, such as, at least about 50 μg/kg, 75 μg/kg, or 90 μg/kg, or at least 160 μg/kg Factor VIIa.

It will be understood that the effective amount of Factor VIIa or Factor VIIa equivalent, as well as the overall dosage regimen, may vary according to the patient's haemostatic status, which, in turn, may be reflected in one or more clinical parameters, including, e.g., relative levels of circulating coagulation factors; amount of blood lost; rate of bleeding; haematocrit, and the like. It will be further understood that the effective amount may be determined by those of ordinary skill in the art by routine experimentation, by constructing a matrix of values and testing different points in the matrix.

For example, in one series of embodiments, the invention encompasses (i) administering a first dose of Factor VIIa or a Factor VIIa equivalent; (ii) assessing the patient's coagulation status after a predetermined time; and (iii) based on the assessment, administering a further dose of Factor VIIa or Factor VIIa equivalent if necessary. Steps (ii) and (iii) may be repeated until satisfactory haemostasis is achieved.

According to the invention, Factor VIIa or a Factor VIIa equivalent may be administered by any effective route, including, without limitation, intravenous, intramuscular, subcutaneous, mucosal, and pulmonary routes of administration. Preferably, administration is by an intravenous route.

*Combination treatments:*

The present invention encompasses combined administration of an additional agent in concert with Factor VIIa or a Factor VIIa equivalent. In some embodiments, the additional agent comprises a coagulant, including, without limitation, a coagulation factor such as, e.g., Factor VIII, Factor IX, Factor V, Factor XI, or Factor XIII; or an inhibitor of the fibrinolytic system, such as, e.g., PAI-1, aprotinin, ε -aminocaproic acid or tranexamic acid.

It will be understood that, in embodiments comprising administration of combinations of Factor VIIa with other agents, the dosage of Factor VIIa or Factor VIIa equivalent may on its own comprise an effective amount and additional agent(s) may further augment the therapeutic benefit to the patient. Alternatively, the combination of Factor VIIa or equivalent and the second agent may together comprise an effective amount for preventing complications associated with ICH. It will also be understood that effective amounts may be defined in the context of particular treatment regimens, including, e.g., timing and number of administrations, modes of administrations, formulations, etc.

### *Treatment outcomes:*

The present invention provides compositions for preventing or attenuating haemorrhage growth, and/or oedema generation following ICH, as well as preventing and/or attenuating one or more complications of ICH. Complications include, without limitation, Cerebral Oedema, decreased quality of life including death caused by one or more of these syndromes.

In practicing the present invention, the severity of ICH and its complications may be assessed using conventional methods, such as, e.g., Imaging by CT or MR scans or the Clinical assessment scores (Scores) described herein. Assessments may be performed at least about 15 days from the start of treatment according to the invention, such as, e.g., at least about 30 days, at least about 40 days, or at least about 90 days from the start of treatment.

Organ damage or organ failure encompasses, without limitation, damage to the structure and/or damage to the functioning of the organ i.e. the brain, defined but not limited to cerebrum, cerebellar, pons, medulla, brain stem, spinal cord or surrounding tissues. Examples of organ damage include, but are not limited to, morphological/structural damage and/or damage to the functioning of the organ such as, for example accumulation of excess fluid or proteins. The terms "organ injury", "organ damage" and "organ failure" may be used interchangeably. Normally, organ damage results in organ failure. By organ failure is meant a decrease in organ function compared to the mean, normal functioning of a corresponding organ in a normal, healthy person. The organ failure may be a minor decrease in function (e.g., 80-90% of normal) or it may be a major decrease in function (e.g., 10-20% of normal); the decrease may also be a complete failure of organ function. Organ failure includes, without limitation, decreased biological functioning, e.g., due to tissue necrosis, fibrin deposition, haemorrhage, oedema, or inflammation and/or the complications that occur in response to these failures like brain herniation. Organ damage includes, without limitation, tissue necrosis, fibrin deposition, haemorrhage, oedema, or inflammation.

Methods for testing organ function and efficiency, and suitable biochemical or clinical parameters for such testing, are well known to the skilled clinician. Such markers or biochemical parameters of organ function are, for example:

Brain perfusion: Measurements of Cerebral blood flow
Brain metabolism: Measurements of cerebral oxygen extraction or direct measurements of cerebral metabolic rate of oxygen (e.g., by MRS, PET or SPECT scans). Measurement of other substrates than oxygen such as glucose are also included.
Brain integrity: MRI (any and all standarized protocol sequences), CT, CTA, MRA Brain cell electrical function as measured by EEG
Brain function by well established neurological tests (e.g., Microdialysis, Transcranial Doppler)

Methods for diagnosing coagulopathy and inflammation are also well known to the skilled clinician. Markers of a coagulapathic state are, for example, PTT, Fibrinogen depletion, elevation in TAT complexes, ATIII activity, IL-6, IL-8, or TNFR-1.

In the present context, prevention includes, without limitation, the attenuation, elimination, minimization, alleviation or amelioration of one or more symptoms or conditions associated with ICH and/or its complications, including, but not limited to, the prevention of further damage to and/or failure of the effected organ already subject to some degree of organ failure and/or damage, as well as the prevention of damage and/or failure of further organs not yet subject to organ failure and/or damage. Examples of such symptoms or conditions include, but are not limited to, morphological/structural damage and/or damage to the functioning of organs such as, but not limited to, brain and surrounding organs.

Examples of such symptoms or conditions include, but are not limited to, morphological/structural damage and/or damage to the functioning of the organ(s) such as, for example, accumulation of proteins or fluids due to mass effect of the haematoma or from resulting inflammatory reactions in the surrounding tissue, tissue necrosis, fibrin deposition, haemorrhage, oedema, or inflammation.

Attenuation of organ failure or damage encompasses any improvement in organ function as measured by at least one of the well known markers of function of said organ (see below) compared to the corresponding value(s) found in ICH patients not being treated in accordance with the present invention.

In various embodiments, haematoma growth is reduced by at least 5%, such as, e.g., 10%, 20%, 30%, 40%, 50%, 60%, or at least 70% compared to haematoma growth in patients not treated with Factor VIIa or a Factor VIIa equivalent in accordance with the present invention. In various embodiments, oedema generation is reduced by at least 5%, such as, e.g., 10%, 20%, 30%, 40%, 50%, 60%, or at least 70% compared to oedema generation in patients not treated with Factor VIIa or a Factor VIIa equivalent in accordance with the present invention. In other embodiments, total haemorrhage volume (ICH+IVH) is reduced by at least 5%, such as, e.g., 10%, 20%, 30%, 40%, 50%, 60%, or at least 70% compared to total haemorrhage volume in patients not treated with Factor VIIa or a Factor VIIa equivalent in accordance with the present invention.

*Measurement of ICH severity and/or Complications:*

Following are non-limiting examples of methods for assessing the incidence and severity of complications of ICH.

The Glasgow Coma Score (GCS; Teasdale and Jennett, The Lancet 13; 2(7872):81-84, 1974) is determined by use of the attached instrument or its equivalents (Appendix 1; below)

The modified Rankin Scale (mRS; Bonita and Beaglehole, Stroke 1988 Dec; 19(12): 1497-1500) is determined by use of the attached instrument or its equivalents (Appendix 2; below)

The Barthel Index (BI; Mahoney and Barthel, Maryland State Medical Journal 1965; 14:56-61) is determined by use of the attached instrument or its equivalents (Appendix 3; below).

The NIH Stroke Scale (NIHSS; Brott et al., 1989) is determined using the attached instrument or its equivalent (Appendix 4; below).

The Glasgow Outcome Scale extended version (GOSe, Lindsay et al., Journal of Neurotrauma; 15 (8): 573-580, 1998) is determined by use of the attached instrument or its equivalents (Appendix 5; below).

Information of scales and assessment tools, including the five above-mentioned well-known instruments, is found at The Internet Stroke Center Washington, University School of Medicine, Department of Neurology (www.strokecenter.org).

*Other indices of treatment:*

The efficacy of the methods of the present invention may also be assessed using other clinical parameters, including, without limitation, reduction in any one or more of the following parameters relative to a similar patient who has not been administered Factor VIIa or a Factor VIIa equivalent according to the invention: an improvement in neurological outcome as determined by the NIHSS, the mRS, the E-GOS, the GCS or the BI scales as described above.; a decrease in the number of days of hospitalization after suffering an ICH, including a decrease in the number of days that a patient may spend in an intensive care unit (ICU) and a decrease in the number of days in which certain interventions (such as, e.g., a ventilator) are required. Non-limiting examples of outcomes include: (i) an improvement in scores on the NIHSS by at least 1, 2 , 4, 8, 10, or 20 points on the scale(ii) an improvement on the mRS scale by at least 1, 2, 3, or 4 points on the scale;(iii) an improvement on the BI scale by at least 5, 10, 15, 20 or 30 points on the scale; (iv) an improvement on the 8 point GOS by at least 1, 2, 3, 5, or 7 points on the scale (v) a decrease in ICU days by 1 day, 2 days, or 4 days; (vi) a reduction on the number of days on a ventilator by 1 day, 2 days, or 4 days; (vii) a reduction in the total days of hospitalization by 2 days, 4 days, or 8 days.

**Various embodiments of the invention**

The present invention encompasses the use of Factor VIIa or a Factor VIIa equivalent for the manufacture of a medicament for preventing or attenuating haemorrhage growth, and/or oedema generation following ICH as well as preventing or attenuating one or more complications of ICH. Non-limiting examples of complications include: cerebral oedema and poor neurological outcome after ICH, and death. In some embodiments, the patient is suffering from spontaneous ICH and in some from traumatic ICH.

In some embodiments, the medicament comprises at least about 160 μg/kg of Factor VIIa or a corresponding amount of a Factor VIIa equivalent. In some embodiments, the medicament is for administration in a first dose containing at least about 160 μg/kg.

In another aspect, the invention encompasses the use of Factor VIIa or a Factor VIIa equivalent for the manufacture of a medicament for reducing the number of days an ICH patient is hospitalized following symptom onset or injury onset. In some embodiments, the medicament is for reducing the number of days an ICH patient spends in an Intensive Care Unit (ICU) following injury or symptom onset.

In another aspect, the invention encompasses the use of Factor VIIa or a Factor VIIa equivalent for the manufacture of a medicament for improving brain function in an ICH patient. In some embodiments, the medicament is for reducing the amount of brain oedema and the associated risks of further neurological deterioration associated with such oedema in an ICH patient. In some embodiments, the medicament is for reducing the risk of progression from brain injury.

In another aspect, the invention encompasses the use of Factor VIIa or a Factor VIIa equivalent for the manufacture of a medicament for reducing the risk of death in an ICH patient.

In some embodiments, the medicament further comprises a second coagulation agent in an amount that augments said preventing or attenuating by the Factor VIIa or Factor VIIa equivalent. In some embodiments, the second coagulation agent is selected from the group consisting of a coagulation factor and an antifibrinolytic agent. Non-limiting examples of a coagulation factor include Factor VIII, Factor IX, Factor V, Factor XI, Factor XIII, and any combination of the foregoing; and non-limiting examples of the antifibrinolytic agent include PAI-1, aprotinin, $\epsilon$ -aminocaproic acid, and tranexamic acid.

In some embodiments, the effective amount comprises at least about 40 $\mu$g/kg of Factor VIIa or a corresponding amount of a Factor VIIa equivalent. In some embodiments, a first amount of at least about 160 $\mu$g/kg Factor VIIa or a corresponding amount of a Factor VIIa equivalent is administered at the start of treatment, and a second amount of about 40, 80 or 160 $\mu$g/kg of Factor VIIa or a corresponding amount of a Factor VIIa equivalent is administered to the patient one hour after the start of treatment.

*The following examples are intended as non-limiting illustrations of the present invention.*

EXAMPLES

*General methods*

**Methods of measurement.**

Haematoma, Intraventricular Haemorrhage (IVH) and oedema volumes ($mm^3$) were measured by computed tomography scan (CT scan) using Analyze™ software (Biomedical Imaging Resource at the Mayo Foundation) equipped with a ROI (region of interest) module.

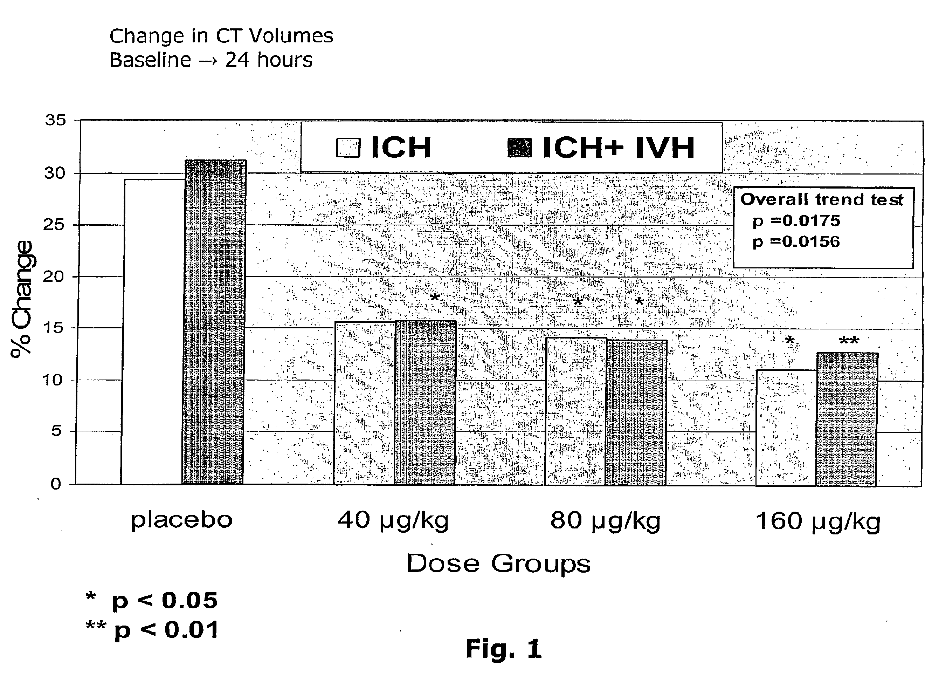

Haematoma, Intraventricular Haemorrhage (IVH) and oedema volumes ($mm^3$) were calculated by a neuroradiologist blinded to treatment allocation at a centralized location by tracing the perimeter of appropriate high- or low-attenuation zone in each involved cross-sectional image ("slice") using a computerized imaging system. All measurements will be made using the Region of Interest (ROI) module within the Analyze™ software. The reviewer will define each target area using the semi-automated segmentation and/or freehand tracing tools. Each defined area will be editable to aid the reviewer to include only the area of interest, which is represented as a ROI on the image (see Figs. 1a and 1b). This procedure will be reproduced for each slice as well as each separate target area the reviewer determines is necessary.

Once all areas have been defined by the reviewer, Analyze™ software will be used to calculate statistics of each ROI. This is to include the area of the ROI, defined in $mm^2$ and the volume of the ROI, defined in $mm^3$. The volume of the ROI is calculated by multiplying the slice thickness of the acquisition by the area. Since an object may include more then one slice, the ROI volume for each slice is displayed in a "Stat Log Region of Interest" window. This information in this window is saved as an ASCII file and directly imported into the Blind Read Database. Once all volumes have been imported into the Blind Read Database, the reviewer-defined regions are stored as object maps and saved. An object map is simply a copy of the volume with defined areas/structures.

The reviewer will use the Region of Interest (ROI) module from the Analyze™ software to measure the following:

- The volume of ICH.
- The volume of Intraventricular Haemorrhage (IVH).
- The total volume of Perihematoma oedema.

Based upon the above measurements the following will be calculated:

- The change in ICH volume from the screening to the 24 hour CT scan expressed in $mm^3$ and percent.

Calculated as change in percent = [(ICH volume at 24 hours - ICH volume at screening)/ICH volume at screening] 100.

Calculated as change in millimeters$^3$ = ICH volume at 24 hours - ICH volume at screening

- Ratio of oedema/ICH volume at each CT scan

Ratio = volume of oedema / ICH volume

- Total Haemorrhage at each CT scan.

Calculated as Total Haemorrhage = ICH + IVH

The following calculations will be used on CT scans submitted using multi-slice thickness techniques. (A smaller slice thickness used through the posterior fossa with a transition to a larger slice thickness through to the vertex.)

1 = Smaller Slice Thickness Acquisition
2 = Larger slice Thickness Acquisition

Calculated as Slice Thickness (mm) / Slice Spacing (mm) = X / [Table Position 1 (mm) + ½ Slice Spacing 1 (mm)] – [Table Position 2 (mm) – ½ Slice Spacing 2 (mm)]
Volume of Gap/Overlap in millimeters$^3$ = X (Area of final slice in 1 in millimeters$^2$)

*Working examples*

**Example 1: Efficacy, of Factor VIIa given to ICH patients**

*OBJECTIVES:*

To evaluate the efficacy and safety of NovoSeven® in preventing early haematoma growth in acute Intracerebral Haemorrhage (ICH). Treatment is intended to be administered as soon as possible following injury or symptom onset but can be delayed due to transportation and/or mitigating medical circumstances. Timing should be in the order of hours not days following onset of injury or symptoms.

*TRIAL DESIGN:*

The trial was a randomised, double-blind, multi-centre, multi-national, placebo-controlled efficacy and safety trial with four treatment arms: doses of 40, 80 and 160μg/kg against placebo.

*TRIAL POPULATION:*

Number of subjects to be studied: 400 (four hundred) patients with ICH.

**Inclusion Criteria**

1. Spontaneous ICH (incl brainstem and cerebellum) diagnosed by CT scanning within 3 hours of onset
2. Male or female subjects, age ≥ 18 years
3. Signed informed consent form, or in countries where waiver of informed consent is allowed by IRB/IEC, a completed waiver form

**Exclusion Criteria**

1. Time of onset of symptoms of ICH unknown or >3 hours
2. Patients with secondary ICH related to infarction, tumour, haemorrhagic infarction, cerebrovenous thrombosis, aneurysm, arteriovenous malformations (AVM), thrombolysis or severe trauma
3. Surgical haematoma evacuation planned or performed within 24 hours of onset
4. Deep Coma (GCS 3-5) at the time of admission
5. Known oral anti-coagulant use (unless INR documented below 1.4). Aspirin use is not an exclusion criterion
6. Known thrombocytopenia (unless platelets documented above $50,000/\mu L$)
7. Preexisting disability (i.e must have mRS score 0-2 before stroke)
8. Any history of haemophilia or other coagulopathy.
9. Acute myocardial ischaemia, acute septicaemia, acute crush injury, acute haemorrhagic disseminated intravascular coagulation, or acute thrombotic stroke.
10. Pregnancy
11. History of angina, myocardial infarction, ischaemic stroke, previous limb amputation due to vascular disease, or claudication within last 30 days.
12. Known or suspected allergy to trial product or related products
13. Previous participation in this trial
14. Participation in ANY investigational drug or device trial within 30 days of entry into the trial

*ASSESSMENTS:*

*Efficacy Endpoints:*

- The primary efficacy endpoint is the change in ICH volume as measured by CT head scans from prior to dosing to 24 hours after the baseline scan.
- Secondary efficacy endpoints are differences between groups on the modified Rankin Scale (mRS), the Barthel Index (BI), the Glasgow Coma Scale (GCS), the 8-point Glasgow Outcome Scale (GOS), The EuroQOL scale, and the National Institute of Health's Stroke Scale (NIHSS) over the duration of the trial.

*Safety Endpoints:*

- The occurrence of adverse events until discharge and serious adverse events until the End of Trial Form is completed
- Safety laboratory coagulation parameters (Fibrinogen and Fragment1+2) from prior to dosing to one hour post-dosing
- Exacerbation of brain oedema (oedema/ICH-volume ratio) as assessed using Head CT scan at 72 hours post-dosing

*TRIAL PRODUCT(S):*

Activated recombinant human factor VII (rFVIIa/NovoSeven®) and placebo are supplied by Novo Nordisk A/S, Denmark as freeze-dried powders to be reconstituted with water for injection.

## Example 2: Factor VIIa administration to ICH patients

The ITT population included 399 patients, after 1 patient was removed after having withdrawn consent. ICH haematoma growth (ITT): % change in baseline to 24 hours

| Placebo | 40 µg/kg | 80 µg/kg | 160 µg/kg | P value |
|---|---|---|---|---|
| 29% | 16% p=0.0710 | 14% p=0.0486 | 11% P=0.0150 | 0.0175 (overall test for trend) 0.0112 (combined NovoSeven® vs placebo) |

Change in CT volumes: % change in baseline to 24 hours (illustrated also in Figure 1)

| Placebo | 40 µg/kg | 80 µg/kg | 160 µg/kg | P value |
|---|---|---|---|---|
| ICH 29% ICH+IVH 31% | ICH 16% ICH+IVH 16% P<0.05 | ICH 14% P<0.05 ICH+IVH 14% P<0.05 | ICH 12% P<0.05 ICH+IVH 13% P<0.01 | Overall trend test: ICH p=0.0175 ICH+IVH p=0.0156 |
| ICH: intracerebral haemorrhage Total Haemorrhage = ICH + IVH | | IVH: intraventricular haemorrhage | | |

**Example 3: Factor VIIa administration to ICH patients**

Secondary Efficacy, Haemorrhage Endpoints Analysis of Edema Volume at 72 Hours after Baseline ITT Population

| | Mean | SE | 98.3% CI | p-value |
|---|---|---|---|---|
| Test for trend | | | | 0.0014 |
| 40 ug/kg - Placebo | -3.90 | 2.51 | [-9.95; 2.15] | 0.1219 |
| 80 ug/kg - Placebo | -6.05 | 2.62 | [-12.35; 0.25] | 0.0216 |
| 160 ug/kg - Placebo | -7.97 | 2.56 | [-14.13;-1.82] | 0.0020 |
| rFVIIa Combined - Placebo | -5.92 | 2.10 | [-10.98;-0.86] | 0.0051 |
| Change in volumes are analysed by a generalised linear mixed model with subject and reader as random effects and volume at baseline, time from stroke to baseline CT, and time from baseline CT to dosing as fixed effects covariates | | | | |

Secondary Efficacy, Haemorrhage Endpoints Analysis of Total Edema Volume (ICH+IVH+Edema) at 72 Hours after Baseline ITT Population

| | Mean | SE | 98.3% CI | p-value |
|---|---|---|---|---|
| Test for trend | | | | 0.0006 |
| 40 ug/kg - Placebo | -6.54 | 4.37 | [-17.06; 3.97] | 0.1351 |
| 80 ug/kg - Placebo | -12.23 | 4.55 | [-23.18;-1.28] | 0.0076 |
| 160 ug/kg - Placebo | -14.41 | 4.45 | [-25.11;-3.72] | 0.0013 |
| rFVIIa Combined - Placebo | -10.92 | 3.66 | [-19.73;-2.12] | 0.0030 |
| Change in volumes are analysed by a generalised linear mixed model with subject and reader as random effects and volume at baseline, time from stroke to baseline CT, and time from baseline CT to dosing as fixed effects covariates | | | | |

**Example 4: Factor VIIa administration to ICH patients**

Patients not developing oedema (oedema volume =0) - change in baseline to 72 hours

| Placebo | 40 μg/kg | 80 μg/kg | 160 μg/kg |
|---------|----------|----------|-----------|
| 3 | 1 | 2 | 7 |
| 1.7% | 0.5% | 1.2% | 3.9% |

All patents, patent applications, and literature references referred to herein are hereby incorporated by reference in their entirety.

Many variations of the present invention will suggest themselves to those skilled in the art in light of the above detailed description. Such obvious variations are within the full intended scope of the appended claims.

*Appendices*

Appendix 1
Glasgow Coma Score (GOS):

Appendix 2
Modified Rankin Scale (mRS):

Appendix 3
Barthel Index (BI):

Appendix 4
NIH Stroke Scale (NIHSS):

Appendix 5
Glasgow Outcome Scale extended version (GOSe or E-GOS):

# GLASGOW
# COMA
# SCALE

Patient Name: _____

Rater Name: _____

Date: _____

## Activity                                                            Score

**EYE OPENING**

| | |
|---|---|
| None | 1 = Even to supra-orbital pressure |
| To pain | 2 = Pain from sternum/limb/supra-orbital pressure |
| To speech | 3 = Non-specific response, not necessarily to command |
| Spontaneous | 4 = Eyes open, not necessarily aware | _____

**MOTOR RESPONSE**

| | |
|---|---|
| None | 1 = To any pain; limbs remain flaccid |
| Extension | 2 = Shoulder adducted and shoulder and forearm internally rotated |
| Flexor response | 3 = Withdrawal response or assumption of hemiplegic posture |
| Withdrawal | 4 = Arm withdraws to pain, shoulder abducts |
| Localizes pain | 5 = Arm attempts to remove supra-orbital/chest pressure |
| Obeys commands | 6 = Follows simple commands | _____

**VERBAL RESPONSE**

| | |
|---|---|
| None | 1 = No verbalization of any type |
| Incomprehensible | 2 = Moans/groans, no speech |
| Inappropriate | 3 = Intelligible, no sustained sentences |
| Confused | 4 = Converses but confused, disoriented |
| Oriented | 5 = Converses and oriented | _____

**TOTAL (3–15):** _____

## References

Teasdale G, Jennett B. "Assessment of coma and impaired consciousness. A practical scale."
*The Lancet* 13;2(7872):81-4, 1974.

# MODIFIED RANKIN SCALE (MRS)

Patient Name: _____

Rater Name: _____

Date: _____

| Score | Description |
|-------|-------------|
| 0 | No symptoms at all |
| 1 | No significant disability despite symptoms; able to carry out all usual duties and activities |
| 2 | Slight disability; unable to carry out all previous activities, but able to look after own affairs without assistance |
| 3 | Moderate disability; requiring some help, but able to walk without assistance |
| 4 | Moderately severe disability; unable to walk without assistance and unable to attend to own bodily needs without assistance |
| 5 | Severe disability; bedridden, incontinent and requiring constant nursing care and attention |
| 6 | Dead |

**TOTAL (0–6):** _____

## References

Rankin J. "Cerebral vascular accidents in patients over the age of 60."
*Scott Med J* 1957;2:200-15

Bonita R, Beaglehole R. "Modification of Rankin Scale: Recovery of motor function after stroke."
*Stroke* 1988 Dec;19(12):1497-1500

Van Swieten JC, Koudstaal PJ, Visser MC, Schouten HJ, van Gijn J. "Interobserver agreement for the assessment of handicap in stroke patients."
*Stroke* 1988;19(5):604-7

# THE BARTHEL INDEX

Patient Name: _____

Rater Name: _____

Date: _____

**Activity**        **Score**

**FEEDING**
0 = unable
5 = needs help cutting, spreading butter, etc., or requires modified diet
10 = independent     \_\_\_\_\_

**BATHING**
0 = dependent
5 = independent (or in shower)     \_\_\_\_\_

**GROOMING**
0 = needs to help with personal care
5 = independent face/hair/teeth/shaving (implements provided)     \_\_\_\_\_

**DRESSING**
0 = dependent
5 = needs help but can do about half unaided
10 = independent (including buttons, zips, laces, etc.)     \_\_\_\_\_

**BOWELS**
0 = incontinent (or needs to be given enemas)
5 = occasional accident
10 = continent     \_\_\_\_\_

**BLADDER**
0 = incontinent, or catheterized and unable to manage alone
5 = occasional accident
10 = continent     \_\_\_\_\_

**TOILET USE**
0 = dependent
5 = needs some help, but can do something alone
10 = independent (on and off, dressing, wiping)     \_\_\_\_\_

**TRANSFERS (BED TO CHAIR AND BACK)**
0 = unable, no sitting balance
5 = major help (one or two people, physical), can sit
10 = minor help (verbal or physical)
15 = independent     \_\_\_\_\_

**MOBILITY (ON LEVEL SURFACES)**
0 = immobile or < 50 yards
5 = wheelchair independent, including corners, > 50 yards
10 = walks with help of one person (verbal or physical) > 50 yards
15 = independent (but may use any aid; for example, stick) > 50 yards     \_\_\_\_\_

**STAIRS**
0 = unable
5 = needs help (verbal, physical, carrying aid)
10 = independent     \_\_\_\_\_

**TOTAL (0–100):**   \_\_\_\_\_

## The Barthel ADL Index: Guidelines

1. The index should be used as a record of what a patient does, not as a record of what a patient could do.
2. The main aim is to establish degree of independence from any help, physical or verbal, however minor and for whatever reason.
3. The need for supervision renders the patient not independent.
4. A patient's performance should be established using the best available evidence. Asking the patient, friends/relatives and nurses are the usual sources, but direct observation and common sense are also important. However direct testing is not needed.
5. Usually the patient's performance over the preceding 24-48 hours is important, but occasionally longer periods will be relevant.
6. Middle categories imply that the patient supplies over 50 per cent of the effort.
7. Use of aids to be independent is allowed.

## References

Mahoney FI, Barthel D. "Functional evaluation: the Barthel Index."
*Maryland State Medical Journal* 1965;14:56-61. Used with permission.

Loewen SC, Anderson BA. "Predictors of stroke outcome using objective measurement scales."
*Stroke.* 1990;21:78-81.

Gresham GE, Phillips TF, Labi ML. "ADL status in stroke: relative merits of three standard indexes."
*Arch Phys Med Rehabil.* 1980;61:355-358.

Collin C, Wade DT, Davies S, Horne V. "The Barthel ADL Index: a reliability study."
*Int Disability Study.* 1988;10:61-63.

## Copyright Information

# EP 1 761 274 B1

**NIH STROKE SCALE**

FORM 5

1 of 4

The NINDS t-PA Stroke Trial No. ___ ___-___ ___ ___-___ ___ ___

Pt. Date of Birth ___ ___/___ ___/___ ___

Hospital _____(___ ___-___ ___)

Date of Exam ___ ___/___ ___/___ ___

Interval:  1[ ] Baseline   2[ ] 2 hours post treatment   3[ ] 24 hours post onset of symptoms ±20 minutes   4[ ] 7-10 days
5[ ] 3 months   6[ ] Other _____(___ ___)

Time: ___ ___:___ ___   1[ ]am  2[ ]pm

Administer stroke scale items in the order listed. Record performance in each category after each subscale exam. Do not go back and change scores. Follow directions provided for each exam technique. Scores should reflect what the patient does, not what the clinician thinks the patient can do. The clinician should record answers while administering the exam and work quickly. Except where indicated, the patient should not be coached (i.e., repeated requests to patient to make a special effort).

**IF ANY ITEM IS LEFT UNTESTED, A DETAILED EXPLANATION MUST BE CLEARLY WRITTEN ON THE FORM. ALL UNTESTED ITEMS WILL BE REVIEWED BY THE MEDICAL MONITOR, AND DISCUSSED WITH THE EXAMINER BY TELEPHONE.**

| Instructions | Scale Definition | Score |
|---|---|---|
| **1a. Level of Consciousness:** The investigator must choose a response, even if a full evaluation is prevented by such obstacles as an endotracheal tube, language barrier, orotracheal trauma/bandages. A 3 is scored only if the patient makes no movement (other than reflexive posturing) in response to noxious stimulation. | 0 = Alert; keenly responsive.<br>1 = Not alert, but arousable by minor stimulation to obey, answer, or respond.<br>2 = Not alert, requires repeated stimulation to attend, or is obtunded and requires strong or painful stimulation to make movements (not stereotyped).<br>3 = Responds only with reflex motor or autonomic effects or totally unresponsive, flaccid, areflexic. | _____ |
| **1b. LOC Questions:** The patient is asked the month and his/her age. The answer must be correct - there is no partial credit for being close. Aphasic and stuporous patients who do not comprehend the questions will score 2. Patients unable to speak because of endotracheal intubation, orotracheal trauma, severe dysarthria from any cause, language barrier or any other problem not secondary to aphasia are given a 1. It is important that only the initial answer be graded and that the examiner not "help" the patient with verbal or non-verbal cues. | 0 = Answers both questions correctly.<br>1 = Answers one question correctly.<br>2 = Answers neither question correctly. | _____ |
| **1c. LOC Commands:** The patient is asked to open and close the eyes and then to grip and release the non-paretic hand. Substitute another one step command if the hands cannot be used. Credit is given if an unequivocal attempt is made but not completed due to weakness. If the patient does not respond to command, the task should be demonstrated to them (pantomime) and score the result (i.e., follows none, one or two commands). Patients with trauma, amputation, or other physical impediments should be given suitable one-step commands. Only the first attempt is scored. | 0 = Performs both tasks correctly<br>1 = Performs one task correctly<br>2 = Performs neither task correctly | _____ |
| **2. Best Gaze:** Only horizontal eye movements will be tested. Voluntary or reflexive (oculocephalic) eye movements will be scored but caloric testing is not done. If the patient has a conjugate deviation of the eyes that can be overcome by voluntary or reflexive activity, the score will be 1. If a patient has an isolated peripheral nerve paresis (CN III, IV or VI) score a 1. Gaze is testable in all aphasic patients. Patients with ocular trauma, bandages, pre-existing blindness or other disorder of visual acuity or fields should be tested with reflexive movements and a choice made by the investigator. Establishing eye contact and then moving about the patient from side to side will occasionally clarify the presence of a partial gaze palsy. | 0 = Normal<br>1 = Partial gaze palsy. This score is given when gaze is abnormal in one or both eyes, but where forced deviation or total gaze paresis are not present.<br>2 = Forced deviation, or total gaze paresis not overcome by the oculocephalic maneuver. | _____ |

17

**NIH STROKE SCALE**

The NINDS t-PA Stroke Trial No. ___ ___-___ ___ ___-___ ___ ___

**FORM 5**

Pt. Date of Birth ___ ___/___ ___/___ ___

2 of 4

Hospital _____(___ ___-___ ___)

Date of Exam ___ ___/___ ___/___ ___

Interval:  1[] Baseline   2[ ] 2 hours post treatment   3[ ] 24 hours post onset of symptoms ±20 minutes   4[ ] 7-10 days
5[] 3 months   6[] Other _____(___ ___)

| | | |
|---|---|---|
| **3. Visual:** Visual fields (upper and lower quadrants) are tested by confrontation, using finger counting or visual threat as appropriate. Patient must be encouraged, but if they look at the side of the moving fingers appropriately, this can be scored as normal. If there is unilateral blindness or enucleation, visual fields in the remaining eye are scored. Score 1 only if a clear-cut asymmetry, including quadrantanopia is found. If patient is blind from any cause score 3. Double simultaneous stimulation is performed at this point. If there is extinction patient receives a 1 and the results are used to answer question 11. | 0 = No visual loss<br><br>1 = Partial hemianopia<br><br>2 = Complete hemianopia<br><br>3 = Bilateral hemianopia (blind including cortical blindness) | _____ |
| **4. Facial Palsy:** Ask, or use pantomime to encourage the patient to show teeth or raise eyebrows and close eyes. Score symmetry of grimace in response to noxious stimuli in the poorly responsive or non-comprehending patient. If facial trauma/bandages, orotracheal tube, tape or other physical barrier obscures the face, these should be removed to the extent possible. | 0 = Normal symmetrical movement<br>1 = Minor paralysis (flattened nasolabial fold, asymmetry on smiling)<br>2 = Partial paralysis (total or near total paralysis of lower face)<br>3 = Complete paralysis of one or both sides (absence of facial movement in the upper and lower face) | _____ |
| **5 & 6. Motor Arm and Leg:** The limb is placed in the appropriate position: extend the arms (palms down) 90 degrees (if sitting) or 45 degrees (if supine) and the leg 30 degrees (always tested supine). Drift is scored if the arm falls before 10 seconds or the leg before 5 seconds. The aphasic patient is encouraged using urgency in the voice and pantomime but not noxious stimulation. Each limb is tested in turn, beginning with the non-paretic arm. Only in the case of amputation or joint fusion at the shoulder or hip may the score be "9" and the examiner must clearly write the explanation for scoring as a "9". | 0 = No drift, limb holds 90 (or 45) degrees for full 10 seconds.<br>1 = Drift, Limb holds 90 (or 45) degrees, but drifts down before full 10 seconds; does not hit bed or other support.<br>2 = Some effort against gravity, limb cannot get to or maintain (if cued) 90 (or 45) degrees, drifts down to bed, but has some effort against gravity.<br>3 = No effort against gravity, limb falls.<br>4 = No movement<br>9 = Amputation, joint fusion explain: _____<br><br>**5a. Left Arm**<br><br>**5b. Right Arm** | _____<br><br>_____ |
| | 0 = No drift, leg holds 30 degrees position for full 5 seconds.<br>1 = Drift, leg falls by the end of the 5 second period but does not hit bed.<br>2 = Some effort against gravity; leg falls to bed by 5 seconds, but has some effort against gravity.<br>3 = No effort against gravity, leg falls to bed immediately.<br>4 = No movement<br>9 = Amputation, joint fusion explain:_____<br><br>**6a. Left Leg**<br><br>**6b. Right Leg** | _____<br><br>_____ |

## NIH STROKE SCALE

FORM 5

3 of 4

The NINDS t-PA Stroke Trial No. ___ ___-___ ___ ___ ___-___ ___ ___

Pt. Date of Birth ___ ___/___ ___/___ ___

Hospital _____(___ ___-___ ___)

Date of Exam ___ ___/___ ___/___ ___

Interval: 1[ ] Baseline   2[ ] 2 hours post treatment   3[ ] 24 hours post onset of symptoms ±20 minutes   4[ ] 7-10 days
5[ ] 3 months   6[ ] Other _____(___ ___)

| | | |
|---|---|---|
| **7. Limb Ataxia:** This item is aimed at finding evidence of a unilateral cerebellar lesion. Test with eyes open. In case of visual defect, insure testing is done in intact visual field. The finger-nose-finger and heel-shin tests are performed on both sides, and ataxia is scored only if present out of proportion to weakness. Ataxia is absent in the patient who cannot understand or is paralyzed. Only in the case of amputation or joint fusion may the item be scored "9", and the examiner must clearly write the explanation for not scoring. In case of blindness test by touching nose from extended arm position. | 0 = Absent<br>1 = Present in one limb<br>2 = Present in two limbs<br><br>If present, is ataxia in<br>Right arm   1 = Yes   2 = No<br>   9 = amputation or joint fusion, explain _____<br>Left arm   1 = Yes   2 = No<br>   9 = amputation or joint fusion, explain _____<br>Right leg   1 = Yes   2 = No<br>   9 = amputation or joint fusion, explain _____<br>Left leg   1 = Yes   2 = No<br>   9 = amputation or joint fusion, explain _____ | _____<br><br><br>_____<br><br>_____<br><br>_____<br><br>_____ |
| **8. Sensory:** Sensation or grimace to pin prick when tested, or withdrawal from noxious stimulus in the obtunded or aphasic patient. Only sensory loss attributed to stroke is scored as abnormal and the examiner should test as many body areas [arms (not hands), legs, trunk, face] as needed to accurately check for hemisensory loss. A score of 2, "severe or total," should only be given when a severe or total loss of sensation can be clearly demonstrated. Stuporous and aphasic patients will therefore probably score 1 or 0. The patient with brain stem stroke who has bilateral loss of sensation is scored 2. If the patient does not respond and is quadriplegic score 2. Patients in coma (item 1a=3) are arbitrarily given a 2 on this item. | 0 = Normal; no sensory loss.<br><br>1 = Mild to moderate sensory loss; patient feels pinprick is less sharp or is dull on the affected side; or there is a loss of superficial pain with pinprick but patient is aware he/she is being touched.<br><br>2 = Severe to total sensory loss; patient is not aware of being touched in the face, arm, and leg. | _____ |
| **9. Best Language:** A great deal of information about comprehension will be obtained during the preceding sections of the examination. The patient is asked to describe what is happening in the attached picture, to name the items on the attached naming sheet, and to read from the attached list of sentences. Comprehension is judged from responses here as well as to all of the commands in the preceding general neurological exam. If visual loss interferes with the tests, ask the patient to identify objects placed in the hand, repeat, and produce speech. The intubated patient should be asked to write. The patient in coma (question 1a=3) will arbitrarily score 3 on this item. The examiner must choose a score in the patient with stupor or limited cooperation but a score of 3 should be used only if the patient is mute and follows no one step commands. | 0 = No aphasia, normal<br><br>1 = Mild to moderate aphasia; some obvious loss of fluency or facility of comprehension, without significant limitation on ideas expressed or form of expression. Reduction of speech and/or comprehension, however, makes conversation about provided material difficult or impossible. For example in conversation about provided materials examiner can identify picture or naming card from patient's response.<br><br>2 = Severe aphasia; all communication is through fragmentary expression; great need for inference, questioning, and guessing by the listener. Range of information that can be exchanged is limited; listener carries burden of communication. Examiner cannot identify materials provided from patient response.<br><br>3 = Mute, global aphasia; no usable speech or auditory comprehension. | _____ |
| **10. Dysarthria:** If patient is thought to be normal an adequate sample of speech must be obtained by asking patient to read or repeat words from the attached list. If the patient has severe aphasia, the clarity of articulation of spontaneous speech can be rated. Only if the patient is intubated or has other physical barrier to producing speech, may the item be scored "9", and the examiner must clearly write an explanation for not scoring. Do not tell the patient why he/she is being tested. | 0 = Normal<br>1 = Mild to moderate; patient slurs at least some words and, at worst, can be understood with some difficulty.<br>2 = Severe; patient's speech is so slurred as to be unintelligible in the absence of or out of proportion to any dysphasia, or is mute/anarthric.<br>9 = Intubated or other physical barrier, explain_____ | _____ |

**NIH STROKE SCALE**

The NINDS t-PA Stroke Trial No. ___ ___-___ ___ ___-___ ___ ___

**FORM 5**

Pt. Date of Birth ___ ___/___ ___/___ ___

4 of 4

Hospital _____(___ ___-___ ___)

Date of Exam ___ ___/___ ___/___ ___

Interval:  1[ ] Baseline   2[ ] 2 hours post treatment   3[ ] 24 hours post onset of symptoms ±20 minutes   4[ ] 7-10 days
5[ ] 3 months   6[ ] Other _____(___ ___)

| | | |
|---|---|---|
| **11.   Extinction and Inattention (formerly Neglect):**   Sufficient information to identify neglect may be obtained during the prior testing. If the patient has a severe visual loss preventing visual double simultaneous stimulation, and the cutaneous stimuli are normal, the score is normal. If the patient has aphasia but does appear to attend to both sides, the score is normal. The presence of visual spatial neglect or anosagnosia may also be taken as evidence of abnormality. Since the abnormality is scored only if present, the item is never untestable. | 0 = No abnormality.<br><br>1 = Visual, tactile, auditory, spatial, or personal inattention or extinction to bilateral simultaneous stimulation in one of the sensory modalities.<br><br>2 = Profound hemi-inattention or hemi-inattention to more than one modality. Does not recognize own hand or orients to only one side of space. | _____ |

Additional item, not a part of the NIH Stroke Scale score.

| | | |
|---|---|---|
| **A. Distal Motor Function:**  The patient's hand is held up at the forearm by the examiner and patient is asked to extend his/her fingers as much as possible.   If the patient can't or doesn't extend the fingers the examiner places the fingers in full extension and observes for any flexion movement for 5 seconds.  The patient's first attempts only are graded.  Repetition of the instructions or of the testing is prohibited. | 0 = Normal (No flexion after 5 seconds)<br><br>1 = At least some extension after 5 seconds, but not fully extended. Any movement of the fingers which is not command is not scored.<br><br>2 = No voluntary extension after 5 seconds.  Movements of the fingers at another time are not scored.<br><br>**a. Left Arm**<br><br>**b. Right Arm** | _____<br><br>_____ |

12. _____   (___ ___ ___)
     Person Administering Scale              Code

You know how.

Down to earth.

I got home from work.

Near the table in the dining room.

They heard him speak on the radio last night.

# MAMA

# TIP – TOP

# FIFTY – FIFTY

# THANKS

# HUCKLEBERRY

# BASEBALL PLAYER

The 8-point GOS (Extended Glasgow Outcome Scale;e-GOS) is determined as follows:

| CONSCIOUSNESS | |
|---|---|
| 1. Is the head injured person able to obey simple commands, or say any words? | 1= No (vegetative state (VS))<br>2=Yes<br>Anyone who shows ability to obey even simple commands, or utter any word or communicate specifically in any other way is no longer considered to be in the vegetative state. Eye movements are not reliable evidence of meaningful responsiveness. Corroborate with nursing staff. Confirmation of VS requires full assessment as in the Royal College of Physician Guidelines |

| INDEPENDENCE IN THE HOME | |
|---|---|
| 2a. Is the assistance of another person at home essential every day for some activities of daily living? | 1= No<br>2=Yes<br>For a 'No' answer they should be able to look after themselves at home for 24 hours if necessary, though they need not actually look after themselves. Independence includes the ability to plan for and carry out the following activities: getting washed, putting on clean clothes without prompting, preparing food for themselves, dealing with callers, and handling minor domestic crises. The person should be able to carry out activities without needing prompting or reminding, and should be capable of being left alone overnight |
| 2b. Do they need frequent help or someone to be around at home most of the time? | 1 = No (Upper SD)<br>2= Yes (Lower SD)<br>For a 'No' answer they should be able to look after themselves at home for up to 8 hours during the day if necessary, though they need not actually look after themselves. |
| 2c. Was assistance at home essential before the injury? | 1 = No<br>2= Yes |
| INDEPENDENCE OUTSIDE THE HOME | |
| 3a. Are they able to shop without assistance? | 1 = No (Upper SD)<br>2= Yes<br>This includes being able to plan what to buy, take care of the money themselves, and behave appropriately in public. They need not normally shop, but must be able to do so. |
| 3b. Were they able to shop without assistance before the injury? | 1 = No<br>2= Yes |
| 4a. Are they able to travel locally without assistance? | 1 = No (Upper SD)<br>2= Yes<br>They may drive or use public transport to get around. Ability to use a taxi is sufficient, provided the person can phone for it themselves and instruct the driver. |
| 4b. Were they able to travel locally without assistance before the injury | 1 = No<br>2= Yes |
| WORK | |
| 5a. Are they currently able to work to their previous capacity? | 1 = No<br>2= Yes<br>If they were working before, then their current capacity of work should be at the same level. If they were seeking work before, then the injury should not have adversely affected their chances of obtaining work or the level of work for which they are eligible. If the patient was a student before injury, then their capacity for study should not have been adversely affected. |

(continued)

| INDEPENDENCE OUTSIDE THE HOME | |
|---|---|
| 5b. How restricted are they? a. Reduced work capacity. b. Able to work only in a sheltered workshop or non-competitive job, or currently unable to work | 1 = a (Upper MD) 2 = b (Lower MD) |
| 5c. Were they either working/ seeking employment before the injury (answer: yes) or were they doing neither (answer: no)? | 1=No 2= Yes |
| SOCIAL & LEISURE ACTIVITIES | |
| 6a. Are they able to resume regular social and leisure activities outside home? | 1=No 2= Yes They need not have resumed all their previous leisure activities, but should not be prevented by physical or mental impairment. If they have stopped the majority of activities because of loss of interest or motivation then this is also considered a disability |
| 6b. What is the extent of restriction on their social and leisure activities? a. Participate a bit less: at least half as often as before injury. b. Participate much less: less than half as often. c. Unable to participate; rarely, if ever, take part | 1= a (Lower GR) 2= b (Upper MD) 3= c (Lower MD |
| 6c. Did they engage in regular social and leisure activities outside home before the injury? | 1=No 2= Yes |
| FAMILY & FRIENDSHIPS | |
| 7a. Have there been psychological problems which have resulted in ongoing family disruption or disruption of friendships? | 1=No 2= Yes Typical post-traumatic personality changes: quick temper, irritability, anxiety, insensitivity to others, mood swings, depression, and unreasonable or childish behaviour |
| 7b. What has been the extent of disruption or strain? a. Occasional - less than weekly b. Frequent - once a week or more, but tolerable c. Constant - daily and intolerable | 1= a (Lower GR) 2= b (Upper MD) 3= c (Lower MD) |
| 7c. Were there problems with family or friends before the injury? | 1=No 2= Yes If there were problems before injury, but these have become markedly worse since injury, then answer "No" to Question 7c |
| RETURN TO NORMAL LIFE | |
| 8a. Are there any other current problems relating to the injury which affect daily life? | 1=No (Upper GR) 2= Yes (Lower GR) Other typical problems reported after head injury: headaches, dizziness, tiredness, sensitivity to noise or light, slowness, memory failures and concentration problems. |

(continued)

| RETURN TO NORMAL LIFE | |
|---|---|
| 8b. Were similar problems present before the injury? | 1=No<br>2= Yes<br>If there were some problems before injury, but these have become markedly worse since injury, then answer "No" to Question 8b |

Scoring: The patient's overall rating is based on the lowest outcome category indicated on the scale:
1. Dead
2. Vegetative state
3. Lower Severe Disability (Lower SD)
4. Upper Severe Disability (Upper SD)
5. Lower Moderate Disability (Lower MD)
6. Upper Moderate Disability (Upper MD)
7. Good Recovery (Lower GR)
8. Upper Good Recovery (Upper GR)

**Claims**

1. Use of a first coagulation agent comprising Factor VIIa or a Factor VIIa equivalent for manufacture of a medicament for preventing or attenuating one or more complications of intracerebral haemorrhage (ICH) in a patient, wherein said patient has not been diagnosed with a congenital bleeding disorder and has not previously been treated with anticoagulation therapies.

2. Use as defined in claim 1, wherein said patient has experienced spontaneous or traumatic ICH.

3. Use as defined in any one of claims 1-2, wherein the administered amount of Factor VIIa or Factor VIIa equivalent comprises at least about 40 $\mu$g/kg.

4. Use as defined in claim 3, wherein the administered amount of Factor VIIa or Factor VIIa equivalent comprises at least about 80 $\mu$g/kg.

5. Use as defined in claim 4, wherein the administered amount of Factor VIIa or Factor VIIa equivalent comprises at least about 120 $\mu$g/kg.

6. Use as defined in any one of claims 1-5, wherein administering of Factor VIIa or Factor VIIa equivalent is carried out within about 24 hours of the occurrence of the ICH.

7. Use as defined in claim 6, wherein said administering is carried out within about 4 hours of the occurrence of the ICH.

8. Use as defined in any one of claims 1-7, wherein the medicament further comprises a second coagulation agent, and wherein the amounts of said first and second coagulation agents together are effective in said preventing or attenuating.

9. Use as defined in claim 8, wherein the second coagulation agent is a coagulation factor.

10. Use as defined in claim 9, wherein said second coagulation agent is selected from the group consisting of: Factor VIII, Factor IX, Factor V, Factor XI, Factor XIII, a Factor VII or Factor VIIa different from the first coagulation agent, and combinations of any of the foregoing.

11. Use as defined in claim 8, wherein said second coagulation agent is an antifibrinolytic agent.

12. Use as defined in claim 11, wherein said antifibrinolytic agent is selected from the group consisting of PAI-1, aprotinin, $\epsilon$ -aminocaproic acid, tranexamic acid, and combinations of any of the foregoing.

13. Use as defined in any one of claims 1-12, wherein said preventing or attenuating results in one or more of: a reduction in the number of days an ICH patient is hospitalized following ICH and a reduction in the risk of death in an ICH patient.

**Patentansprüche**

1. Verwendung eines ersten Gerinnungsmittels, umfassend Faktor VIIa oder ein Faktor-VIIa-Äquivalent, zur Herstellung eines Medikaments zum Verhindern oder Abschwächen von einer oder mehreren Komplikationen von intrazerebraler Blutung (intracerebral haemorrhage; ICH) bei einem Patienten, wobei bei dem Patienten keine kongentiale Blutungsstörung diagnostiziert worden ist und der im Vorfeld nicht mit Antigerinnungstherapien behandelt worden ist.

2. Verwendung wie in Anspruch 1 definiert, wobei der Patient eine spontane oder traumatische ICH durchgemacht hat.

3. Verwendung wie in einem der Ansprüche 1-2 definiert, wobei die verabreichte Menge an Faktor VIIa oder Faktor-VIIa-Äquivalent mindestens etwa 40 $\mu$g/kg umfasst.

4. Verwendung wie in Anspruch 3 definiert, wobei die verabreichte Menge an Faktor VIIa oder Faktor-VIIa-Äquivalent mindestens etwa 80 $\mu$g/kg umfasst.

5. Verwendung wie in Anspruch 4 definiert, wobei die verabreichte Menge an Faktor VIIa oder Faktor-VIIa-Äquivalent mindestens etwa 120 $\mu$g/kg umfasst.

6. Verwendung wie in einem der Ansprüche 1-5 definiert, wobei die Verabreichung von Faktor VIIa oder Faktor-VIIa-Äquivalent innerhalb von etwa 24 Stunden nach Auftreten der ICH durchgeführt wird.

7. Verwendung wie in Anspruch 6 definiert, wobei die Verabreichung innerhalb von etwa 4 Stunden nach Auftreten der ICH durchgeführt wird.

8. Verwendung wie in einem der Ansprüche 1-7 definiert, wobei das Medikament ferner ein zweites Gerinnungsmittel umfasst und wobei die Mengen des ersten und des zweiten Gerinnungsmittels zusammen bei dem Verhindern oder Abschwächen wirksam sind.

9. Verwendung wie in Anspruch 8 definiert, wobei es sich bei dem zweiten Gerinnungsmittel um einen Gerinnungsfaktor handelt.

10. Verwendung wie in Anspruch 9 definiert, wobei das zweite Gerinnungsmittel ausgewählt ist aus der Gruppe, bestehend aus: Faktor VIII, Faktor IX, Faktor V, Faktor XI, Faktor XIII, einem Faktor VII oder Faktor VIIa, der sich von dem ersten Gerinnungsmittel unterscheidet, und Kombinationen von beliebigen des Vorstehenden.

11. Verwendung wie in Anspruch 8 definiert, wobei es sich bei dem zweiten Gerinnungsmittel um ein antifibrinolytisches Mittel handelt.

12. Verwendung wie in Anspruch 11 definiert, wobei das antifibrinolytische Mittel ausgewählt ist aus der Gruppe, bestehend aus PAI-1, Aprotinin, $\varepsilon$-Aminocapronsäure, Tranexaminsäure und Kombinationen von beliebigen des Vorstehenden.

13. Verwendung wie in einem der Ansprüche 1-12 definiert, wobei das Verhindern oder Abschwächen zu einem oder mehreren von Folgendem führt: einer Reduktion der Anzahl von Tagen, an welchen der ICH-Patient nach einer ICH im Krankenhaus verbringt, und einer Reduktion des Todesrisikos bei einem ICH-Patienten.

**Revendications**

1. Utilisation d'un premier agent de coagulation comprenant le facteur VIIa ou un équivalent du facteur VIIa pour la fabrication d'un médicament pour empêcher ou atténuer une ou plusieurs complications de l'hémorragie intracérébrale (HIC) chez un patient, lorsque ledit patient n'a pas fait l'objet d'un diagnostic de trouble de saignement congénital et n'a pas été précédemment traité par des thérapies anticoagulantes.

**2.** Utilisation telle que définie dans la revendication 1, dans laquelle ledit patient a présenté une HIC spontanée ou traumatique.

**3.** Utilisation telle que définie dans l'une des revendications 1 ou 2, dans laquelle la quantité administrée de facteur VIIa ou d'équivalent du facteur VIIa comprend au moins environ 40 µg/kg.

**4.** Utilisation telle que définie dans la revendication 3, dans laquelle la quantité comprend au moins environ 80 µg/kg.

**5.** Utilisation telle que définie dans la revendication 4, dans laquelle la quantité comprend au moins environ 120 µg/kg.

**6.** Utilisation telle que définie dans l'une des revendications 1 à 5, dans laquelle l'administration du facteur VIIa ou de l'équivalent du facteur VIIa est effectuée dans les 24 heures environ de la survenue de l'HIC.

**7.** Utilisation telle que définie dans la revendication 6, dans laquelle ladite administration est effectuée dans les 4 heures environ de la survenue de l'HIC.

**8.** Utilisation telle que définie dans l'une des revendications 1 à 7, dans laquelle le médicament comprend en outre un second agent de coagulation, et dans laquelle les quantités desdits premier et second agents de coagulation pris ensemble sont efficaces pour ladite prévention ou atténuation.

**9.** Utilisation telle que définie dans la revendication 8, dans laquelle le second agent de coagulation est un facteur de coagulation.

**10.** Utilisation telle que définie dans la revendication 9, dans laquelle ledit second agent de coagulation est choisi dans le groupe formé par : facteur VIII, facteur IX, facteur V, facteur XI, facteur XIII, un facteur VII ou un facteur VIIa différent du premier agent de coagulation, et les combinaisons de n'importe lesquels des précédents.

**11.** Utilisation telle que définie dans la revendication 8, dans laquelle ledit second agent de coagulation est un agent antifibrinolytique.

**12.** Utilisation telle que définie dans la revendication 11, dans laquelle ledit agent antifibrinolytique est choisi dans le groupe formé par le PAI-1, l'aprotinine, l'acide ε-aminocaproïque, l'acide tranexamique, et les combinaisons de n'importe lesquels des précédents.

**13.** Utilisation telle que définie dans l'une des revendications 1 à 12, dans laquelle ladite prévention ou atténuation résulte d'une ou plusieurs parmi : une diminution du nombre de jours d'hospitalisation d'un patient HIC après une HIC et une réduction du risque de décès chez un patient HIC.

Change in CT Volumes
Baseline → 24 hours

Fig. 1

* p < 0.05
** p < 0.01

**REFERENCES CITED IN THE DESCRIPTION**

**Patent documents cited in the description**

- US 4784950 A
- US 5997864 A
- US 4456591 A, Thomas
- US 4837028 A
- US 4501728 A
- US 4975282 A

**Non-patent literature cited in the description**

- **Persson et al.** *J. Biol. Chem.,* 1997, vol. 272, 19919-19924
- **Persson.** *FEBS Letts.,* 1997, vol. 413, 359-363
- **Wakabayashi et al.** *J. Biol. Chem.,* 1986, vol. 261, 11097
- **Thim et al.** *Biochem.,* 1988, vol. 27, 7785
- **Scopes.** Protein Purification. Springer-Verlag, 1982
- Protein Purification. VCH Publishers, 1989
- **Osterud et al.** *Biochem.,* 1972, vol. 11, 2853
- **Hedner et al.** *J. Clin. Invest.,* 1983, vol. 71, 1836
- **Teasdale ; Jennett.** *The Lancet,* 1974, vol. 2 (7872), 81-84
- **Bonita ; Beaglehole.** *Stroke,* December 1988, vol. 19 (12), 1497-1500
- **Mahoney ; Barthel.** *Maryland State Medical Journal,* 1965, vol. 14, 56-61
- **Lindsay et al.** *Journal of Neurotrauma,* 1998, vol. 15 (8), 573-580